# EUROPÄISCHE PATENTANMELDUNG

(11) **EP 1 760 055 A2**
(43) Veröffentlichungstag der Anmeldung: **07.03.2007**
(21) Anmeldenummer: 06016610.5
(22) Anmeldetag: 09.08.2006
(51) Int. Cl.: C07B 41/12, C07C 67/30

(54) **Eisen-katalysierte allylische Alkylierung**

(30) Priorität: 26.08.2005 DE 102005040752
(71) Anmelder: Universität Dortmund, 44227 Dortmund (DE)
(72) Erfinder: Plietker, Bernd, 45307 Essen (DE)
(74) Vertreter: Isfort, Olaf

(57) **Zusammenfassung**

Die Erfindung betrifft ein Verfahren zur Durchführung einer Eisen-katalysierten allylischen Alkylierung umfassend
- die Herstellung eines Reaktionsgemisches erhältlich aus
(i) einem allylischen Substrat mit dem Strukturelement C=C-C-X, wobei X eine Abgangsgruppe umfasst, welche ein Carbonat darstellt,
(ii) einem aktiven Fe(-Ii)-Katalysatorkomplex,
(iii) mindestens einem Ligand,
(iv) mindestens einem Lösungsmittel und
(v) einem Nucleophil oder Pronucleophil.

## Beschreibung

Die Erfindung betrifft ein Verfahren zur Durchführung einer Eisen-katalysierten allylischen Alkylierung und dessen Verwendung.

Bei der Synthese von Naturstoffen oder pharmakologisch interessanten Verbindungen werden in der synthetischen organischen Chemie verschiedene Methoden entwickelt, die den Aufbau chiraler Strukturen ermöglichen. Zur Steigerung der Aktivität und Selektivität sind zahlreiche Methoden entwickelt worden. Hierzu zählen beispielsweise die Racematspaltung mittels reversibler Derivatisierung mit chiralen Hilfsstoffen und anschließender Trennung, die Verwendung von enantionmerenreinen Ausgangsstoffen, die Verwendung von chiralen Hilfsstoffen in stöchiometrischen Reaktionen oder der Einsatz von Reaktionen in Gegenwart eines chiralen Katalysators.

Zu den bedeutendsten katalytischen C,C-Bindungsbildungsreaktionen der organischen Chemie zählt die Übergangsmetall-katalysierte, allylische Alkylierung. Dabei wird durch oxidative Addition eines Übergangsmetallkomplexes an ein Allylsubstrat ein Allylkomplex generiert, der mit einem Nucleophil reagiert. Das Übergangsmetall wird wieder reduziert und das Reaktionsprodukt freigesetzt.

Die Darstellung allylisch hoch substituierter Kohlenstoffbausteine stellt jedoch ein zentrales Problem der präparativen synthetischen organischen Chemie dar. Denn in der Regel erfolgt die Alkylierung am weniger hoch substituierten Kohlenstoff. Ein Angriff am sterisch günstigen, unsubstituierten Allylterminus würde zum unerwünschten, linearen Isomer führen. Es ist daher von besonderem Interesse, den Angriff an das höher substituierte Allylende zu lenken.

Zahlreiche Übergangsmetalle können genutzt werden, um gezielt C-Nucleophile mit allylisch-aktivierten Substraten zur Reaktion zu bringen, wobei die organischen Teile der entstehenden Allylmetallfragmente formal als Carbokationenäquivalente betrachtet werden können. Die bislang bekannten Methoden zur Metall-katalysierten allylischen Alkylierung unter Verwendung von Palladium, Nickel, lridium, Rhodium oder ähnlichen Metallen verlaufen über die Bildung intermediärer Metall-Allyl-Komplexe. Zu den bekanntesten und am rasantesten entwickelten Methoden gehört die asymmetrische Palladium-katalysierte allylische Alkylierung nach *TROST et. al.* Zahlreiche Reviews liefern einen guten Überblick über deren Forschung und Anwendung.

Bei der Übergangsmetallkatalyse werden intermediär (π-Allyl)Metall-Komplexe gebildet, die zwar Vorteile im Hinblick auf eine mögliche Stereoinduktion durch chirale Liganden bieten, jedoch häufig im Falle unsymmetrisch substituierter Substrate zur Bildung von Regioisomerengemischen führen. Dadurch ist es häufig nicht möglich, an den unterschiedlich substituierten Termini des Allylfragments eine Steuerung der Regioselektivität der Alkylierung zu erreichen.

Dieser häufig nicht erwünschte Nebeneffekt kann durch die Verwendung von Liganden teilweise abgeschwächt werden. Einige der Systeme führen zwar zu einer Anreicherung der schlecht zugänglichen höheren Verzweigungsprodukte, jedoch sind die Regioselektivitäten nicht zufrieden stellend.

Hohe Regioselektivitäten spielen bei Naturstoffsynthesen (beispielsweise bei der Kohlenhydrat-Synthese) eine große Rolle. Es besteht daher der Bedarf nach einer Methode zur selektiven allylischen Alkylierung, in der die neue C,C-Bindung an dem Kohlenstoff aufgebaut wird, der zuvor mit dem Nucleofug substituiert wurde.

Des Weiteren zeichnen sich die aus dem Stand der Technik bekannten Katalysatoren, zumeist enthalten sie Edelmetalle, durch ihren hohen Preis sowie ihre Empfindlichkeit gegenüber Sauerstoff und Wasser aus. Derartige Reaktionen müssen in der Regel unter striktem Ausschluss von Sauerstoff und Wasser durchgeführt werden, was gerade aus industrieller Sicht mit einem zusätzlichen apparativen Aufwand verbunden ist. Die verwendeten Übergangsmetalle sind zudem toxisch, was aus ökonomischer und ökologischer Sicht besonders problematisch ist. Aufbereitung und Entsorgung toxischer Katalysatoren bedeuten zusätzliche Kosten durch notwendige Sicherheitsmaßnahmen und Aufreinigungsschritte.

Die Entwicklung ungiftiger, preisgünstiger Katalysatoren, die sich durch ein hohes Maß an Regioselektivität in der allylischen Alkylierung auszeichnen, ist daher von großem synthetischem Interesse.

Aus dem Stand der Technik ist ein weiterer Katalysator bekannt, welcher für die allylische Alkylierung eingesetzt werden kann. Bereits 1979 veröffentlichte Roustan die Synthese eines Eisen-Komplexes, der die allylische Alkylierung katalysiert. Dieses System wurde später (erschienen im Jahre 1987) von Zhou durch Verwendung des formalen Fe(-II)-Komplexes [Bu₄N][Fe(CO)₃(NO)] weiterentwickelt. Beide Katalysesysteme zeichnen sich durch eine hohe Katalysatorladung aus. Die allylische Alkylierung erfolgt hierbei nicht unter Ausbildung einer (π-Allyl)Metall- Spezies.

Aufgrund der schlechten Reproduzierbarkeit der Ergebnisse sowie der mangelhaften Beschreibung der Darstellung des aktiven Katalysators wurde dieser Fe(-II)-Komplex nicht weiter entwickelt. Des Weiteren wird die Reaktion dort unter giftiger CO-Atmosphäre durchgeführt. Die Handhabung mit Kohlenmonoxid ist ebenfalls nicht von Vorteil, so dass in diesem Falle keine weitere wissenschaftliche Forschung betrieben wurde.

Es ist daher wünschenswert, ein Verfahren unter Verwendung eines Fe(-II)-Komplexes für die allylische Alkylierung derart weiterzuentwickeln, das die vorgenannten Nachteile eliminiert werden, und aus ökonomischer und ökologischer Sicht eine großtechnische Verwendung möglich wird.

Zur Lösung der Aufgabe schlägt die Erfindung, ausgehend von einem Verfahren der eingangs genannten Art vor, dass ein Reaktionsgemisch erhältlich aus
(i) einem allylischen Substrat mit dem Strukturelement C=C-C-X, wobei X eine Abgangsgruppe umfasst, welche ein Carbonat darstellt,
(ii) einem aktiven Fe(-II)-Katalysatorkomplex,
(iii) mindestens einem Ligand,
(iv) mindestens einem Lösungsmittel und
(v) einem Nucleophil oder Pronucleophil
hergestellt wird.

Bei der Verwendung von Carbonaten als Nucleofug (Abgangsgruppe) wird in situ eine aktive Base erzeugt, welche zur Deprotonierung des Pronucleophils genutzt wird. Die Verwendung eines Carbonats als Abgangsgruppe ermöglicht erfindungsgemäß eine salzfreie Reaktionsführung, was gerade für Anwendungen im präparativen Maßstab sinnvoll ist.

Bevorzugt umfasst das Carbonat das Strukturelement OC(O)OR, wobei R unverzweigte oder verzweigte, substituierte oder unsubstituierte Kohlenwasserstoffe darstellt. Insbesondere kommen Alkylgruppen, wie z.B. Methyl-, Ethyl-, Propyl-, Butyl-, und/oder Arylgruppen in Frage. Vorzugsweise ist R ein *iso*-Butyl. Dieser Umstand ist vor allem für eine industrielle Anwendung von großer Bedeutung, da die Nebenprodukte der Reaktion, nämlich das CO₂ und ein Alkohol R-OH, untoxisch und leicht zu entfernen sind.

Der Fe(-II)-Katalysatorkomplex hat den Vorteil, dass dieser preiswerter als alle aus dem Stand der Technik bekannten Edelmetall-Katalysatoren ist.

Es wird ein Eisen-Katalysatorkomplex des Typs [Kation][Fe(CO)₃(NO)], vorzugsweise des Typs [R₄N][Fe(CO)₃(NO)], besonders bevorzugt der Metallkomplex [Bu₄N][Fe(CO)₃(NO)] nach Zhou, verwendet. Dieser ist lange lagerfähig und leicht einsetzbar. Beliebige Kationen können eingesetzt werden.

Die Herstellung des Eisen-Katalysatorkomplexes nach Xu und Zhou aus J. Org. Chem. 1987, 52, 974 - 977, gelingt nur in sehr schlechten Ausbeuten. Die verbesserte Vorschrift von Yoshio Otusji aus Bull. Chem. Soc. Jpn. 1991, 64, 2965 ― 2977, ausgehend von Fe(CO)₅, liefert höhere Ausbeuten. Jedoch ist die Aktivität des Eisen-Komplexes nicht zufrieden stellend.

Abweichend von der Literaturvorschrift, durch die der Eisen-Katalysatorkomplex, nur in Form eines katalytisch inaktiven öligen Rohproduktes erhältlich ist, wird deshalb erfindungsgemäß eine Umkristallisation aus Methanol und Wasser angeschlossen. Dieses verbesserte Verfahren zur Darstellung eines katalytisch aktiven Eisen-Katalysatorkomplexes wird im Beispiel näher erläutert. Unter Nutzung dieses optimierten Verfahrens wird der Eisen-Katalysator-Komplex in Form eines gelben Feststoffes erhalten, der die für die Reaktion notwendige Aktivität und Reinheit von mindestens 80 % besitzt. Der Eisen-Katalysatorkomplex ist luft- und wasserstabil; er kann in großen Mengen dargestellt und über längere Zeit ohne Aktivitätsverlust gelagert werden.

Tertiäre Phosphine als Übergangsmetall-Liganden sind aus dem Stand der Technik aufgrund zahlreicher spezifischer Eigenschaften bekannt. Sie zeichnen sich durch die Fähigkeit zur Stabilisierung tiefer Oxidationsstufen des Metallzentrums aus. Im Übergangsmetall-Phosphin-Komplexen liegen die Metallzentren meistens in diskreter molekularer Form vor. Diese Verbindungen sind oft in einem weiten Bereich in organischen Lösungsmitteln löslich. Die Variation der Substituenten in PR₃ erlaubt das Einstellen der elektronischen und sterischen Eigenschaften des Liganden und damit auch des Metallzentrums.

Vorzugsweise werden monodenate Liganden, besonders bevorzugt basische Phosphine eingesetzt. Sie wirken reaktivitätssteigernd und können die Katalysatorstandzeit erhöhen.

Besonders bevorzugt wird der Katalysator durch Zugabe von Triphenylphosphin als Liganden in situ derivatisiert. Durch die Zugabe von Phosphin besitzt der Katalysator eine wesentlich höhere Stabilität, wodurch auf die Verwendung einer toxischen CO-Atmosphäre während der gesamten Reaktion verzichtet werden kann.

Es ist vorstellbar, dass während der Alkylierung ein neuer aktiver Eisen-Katalysatorkomplex sich mit dem Phosphin bildet, welcher isoliert und charakterisiert werden kann. Dieser neue Katalysator ist für viele Anwendungsbereiche einsetzbar.

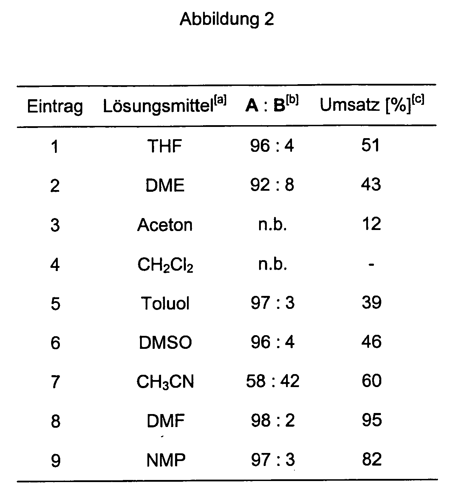

Ein weiterer, oft unterschätzter Punkt ist die Wahl des Lösungsmittels. Es wurde festgestellt, dass stark koordinierende Solventien wie N,N-Dimethylformamid eine weitere Reaktivitätssteigerung ermöglichen. Andere mögliche Lösungsmittel sind in den Beispielen angegeben (Fig. 2).

Ein weiterer Vorteil gegenüber dem Stand der Technik ist die Reduzierung der notwendigen Katalysatormenge von 25 Mol% auf bis zu 2,5 Mol%.

Die Eisen-katalysierte allylische Alkylierung ist auf eine Vielzahl von unterschiedlichen C-Nucleophilen oder Pronuclophilen anwendbar. Vorzugsweise sind diese CH-acid. Nitrilhaltige Nucleophile oder Pronucleophile erweisen sich als besonders reaktiv. Bei deren Anwesenheit verkürzt sich die Reaktionszeit um den Faktor 2.

Vorzugsweise können ebenfalls N-, O- und S-Nucleophile oder Pronucleophile eingesetzt werden.

Die erfindungsgemäße Eisen-katalysierte allylische Alkylierung stellt daher ein einfaches und neues Verfahren zur gezielten C,C-Bindungsbildung ausgehend von Allylcarbonaten dar. Die Alkylierung erfolgt ausschließlich an dem Kohlenstoff des Allylfragments, der zuvor mit der Abgangsgruppe, dem Carbonat, substituiert war.

Ein weiterer Aspekt der Erfindung ist die Verwendung des erfindungsgemäßen Verfahrens für die Synthese von Naturstoffen, Wirkstoffen und nicht natürlichen Aminosäuren. Eine Verwendung ist auch in der asymmetrischen Olefinierung vorstellbar.

Nachstehend wird anhand von Beispielen das erfindungsgemäße Verfahren näher erläutert. Es zeigen:
- Abbildung 1: Ligandeneinfluss in der allylischen Alkylierung
- Abbildung 2: Lösungsmitteleinfluss in der allylischen Alkylierung
- Abbildung 3: Allylische Alkylierung unterschiedlicher Nucleophile
- Abbildung 4: Allylische Alkylierung unterschiedlicher Substrate
- Abbildung 5: Regioselektivität und Stereoselektivität Eisen-katalysierter allylischer Alkylierungen
- Abbildung 6: Konservierung der Regio- und Stereoselektivität im (η¹-Allyl)-Mechanismus
- Abbildung 7: Allylische Alkylierung als Schlüsselprozess in der organischen Chemie

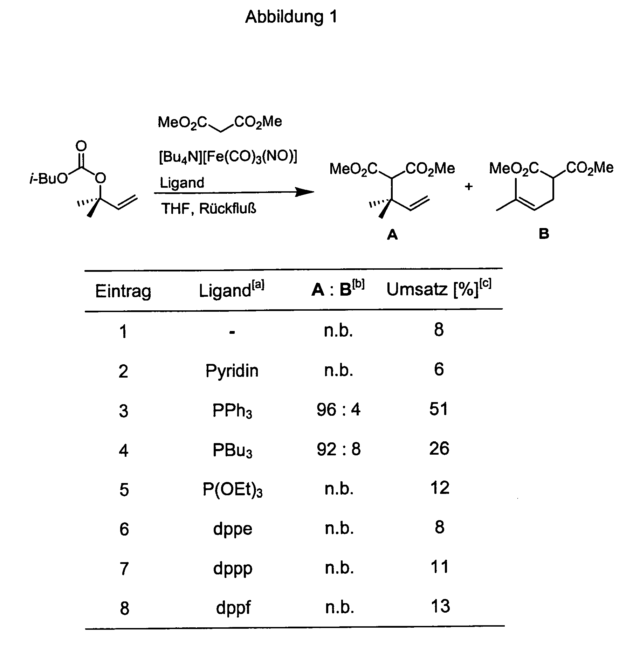

Abbildung 1 stellt eine Reaktionsgleichung dar. In der aufgeführten Tabelle werden zahlreiche Liganden aufgeführt. Dabei zeigen PPh₃ und PBu₃ eine höhere Regioselektivität als die anderen Liganden.

[a] Alle Reaktionen wurden im 1-mmol-Maßstab in Gegenwart von 10 Mol% Fe-Katalysator, 10 Mol% Liganden und 2 Äquivalent des Nucleophils in 5 mL (abs.) THF durchgeführt und nach 24 h gestoppt; [b] Bestimmt via GC; [c] Bestimmt via GC relativ zu Undecan als interner Standard.

Abbildung 2 zeigt [a] Alle Reaktionen, welche in Gegenwart von 10 Mol% PPh₃ unter den in Fig. 1 beschriebenen Bedingungen durchgeführt wurden; [b] Bestimmt via GC; [c] Bestimmt via GC relativ zu Undecan als interner Standard.

Bei der Verwendung von Acetonitril wurde keine große Regioselektivität festgestellt. Dies deutet auf einen alternativen Allyl-Metall-Mechanismus hin.

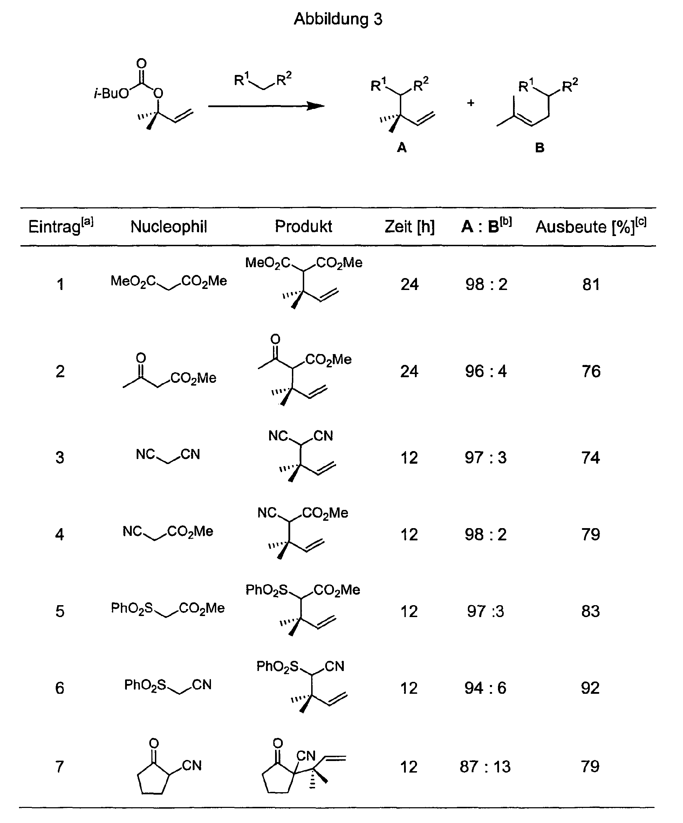

Abbildung 3 zeigt eine Reaktionsgleichung der allylischen Alkylierung und eine Tabelle der möglichen Nucleophile. Die Bandbreite von R¹ und R² sind nahezu grenzenlos.

[a] Alle Reaktionen wurden im 1-mmol-Maßstab in Gegenwart von 2.5 Mol% Fe-Kat., 3 Mol% PPh₃ und 2 Äquivalent des Nucleophils in 1 mL (abs.) DMF durchgeführt; [b] Bestimmt via GC; [c] Bestimmt via GC relativ zu Undecan als interner Standard.

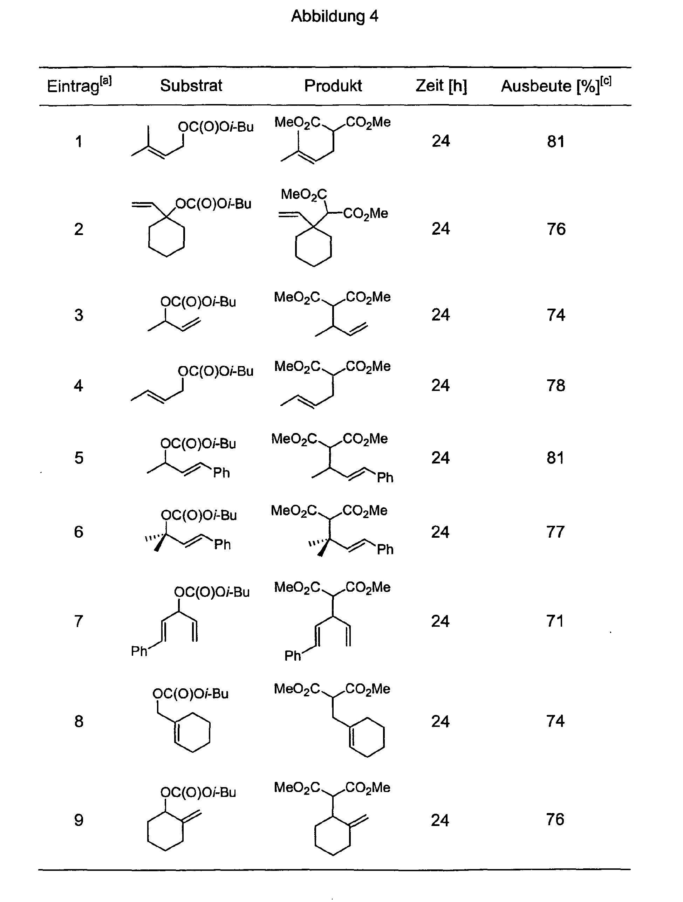

Abbildung 4 zeigt eine Tabelle von möglichen Substraten.

[a] Alle Reaktionen wurden im 1-mmol-Maßstab in Gegenwart von 2.5 Mol% Fe-Kat., 3 Mol% PPh₃ und 2 Äquivalent des Nucleophils in 1 mL (abs.) DMF durchgeführt; [b] Bestimmt via GC; [c] Bestimmt via GC relativ zu Undecan als interner Standard.

Durch die Erhöhung der Substratkonzentration von ursprünglich 0.2 mol/l auf 1 mol/l gelang eine weitere Reaktivitätssteigerung und damit verbunden eine Reduktion der Katalysatorkonzentration auf 2.5 Mol% bei gleich bleibend hoher Regioselektivität.

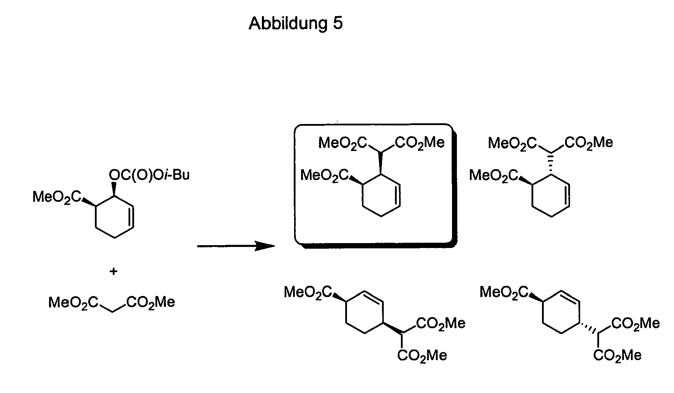

Abbildung 5 zeigt die Regioselektivität der erfindungsgemäßen Alkylierung. Diese legt den Schluss nahe, dass in dieser Reaktion, anders als in den meisten gängigen allylischen Alkylierungen, kein (η³-Allyl)-lntermediat durchlaufen wird. Nur eine hoch stereoselektive doppelte S_{N}2'-anti-Addition sowohl des Metalls im ersten als auch des Nucleophils im zweiten Schritt ermöglicht die beobachtete hohe Regio- und Stereoselektivität.

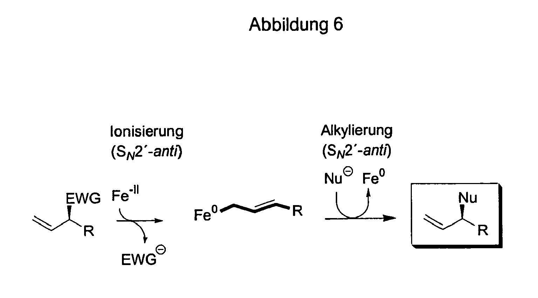

Abbildung 6 zeigt eine mögliche doppelte S*_{N}*2'-anti-Addition-Darstellung.

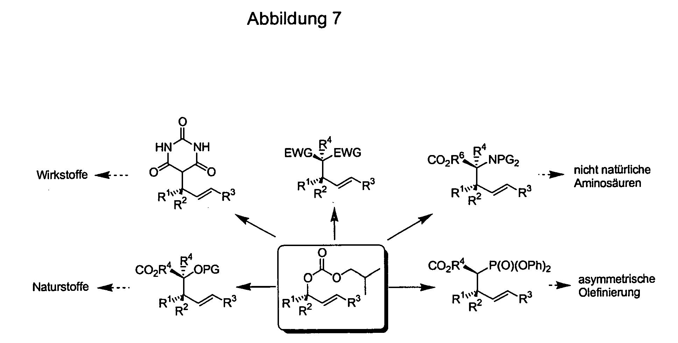

Abbildung 7 zeigt einige Beispiele für die Verwendung der erfindungsgemäßen Alkylierung.

### Beispiel 1

Herstellung von 2-(1',1'-Dimethyl-allyl)-malonsäuredimethylester.

In einem 10-ml-Schlenkrohr werden unter Argon der Eisen-Katalysator (10.2 mg, 0.025 mmol, 2.5 Mol%) und PPh₃ (6.9 mg, 0.03 mmol, 3 Mol%) in trockenem DMF (1 ml) vorgelegt. Das Allylcarbonat (186 mg, 1 mmol) und Malonsäuredimethylester (232 mg, 2 mmol) werden zugegeben. Anschließend wird das Gemisch im verschlossenen Schlenkrohr für 24 h auf 80 °C erwärmt. Nach dem Abkühlen auf Raumtemperatur wird mit Diethylether (20 ml) verdünnt, anschließend nacheinander mit Wasser (10 ml), 2 N NaOH-Lösung (10 ml) und Wasser (10 mL) gewaschen. Die Trocknung der vereinigten organischen Phasen erfolgt über einem 1:1-Gemisch von Na₂SO₄ und Aktivkohle (ca 5 g). Nach Filtration und Einengen erhält man eine schwach-gelbe Flüssigkeit, die säulenchromatographisch gereinigt wird (*i*-Hexan/Diethylether, 5:1). Ausbeute: 162 mg (0.81 mmol, 81 %)

### Beispiel 2

### Darstellung des aktiven Eisen-Katalysatorkomplexes [Bu₄N](Fe(CO)₃(NO)]

Unter Argon wird eine Lösung von Fe(CO)₅ (11.8 g, 60 mmol) in entgastem CH₂Cl₂ (20 ml) zu einer Lösung von NaNO₂ (4.2 g, 60 mmol) und tetra-n-Butylammoniumbromid (19 g, 60 mmol) in entgastem Wasser (20 ml) bei Raumtemperatur zugetropft. Das Gemenge wird für vier Stunden bei dieser Temperatur gerührt. Die organische Phase wird abgetrennt und zweimal mit Wasser (20 ml) gewaschen. Das Lösungsmittel der organischen Phase wird ohne Trocknung über Na₂SO₄ im Vakuum bei 30°C Badtemperatur entfernt. Der ölige, tiefbraune Rückstand wird mit Methanol (20 ml) aufgenommen und erneut im Vakuum aufkonzentriert. Dieser Vorgang wird solange wiederholt, bis das Destillat farblos ist. Anschließend versetzt man das braune Rohprodukt mit wenig Methanol und tropft unter kräftigem Rühren Wasser (200 ml) hinzu. Nach ca. 30 min scheidet sich der Katalysator in Form eines gelben Feststoffs ab. Dieser kann an der Luft abfiltriert werden. Zur Trocknung wird der Feststoff in wenig (abs.) CH₂Cl₂ aufgenommen und noch vorhandene Wasserrückstände durch azeotrope Destillation im Vakuum entfernt. Der tiefgelbe Feststoff wird im Hochvakuum getrocknet und kann ohne weitere Reinigungsschritte direkt in der Katalyse verwendet werden. Unter Argon ist der Komplex bei -20 °C über Wochen ohne Aktivitätsverlust lagerbar. Ausbeute: 22.7 g (55.2 mmol, 92 %).

## Patentansprüche

1. Verfahren zur Durchführung einer Eisen-katalysierten allylischen Alkylierung umfassend
- die Herstellung eines Reaktionsgemisches erhältlich aus
(i) einem allylischen Substrat mit dem Strukturelement C=C-C-X, wobei X eine Abgangsgruppe umfasst, welche ein Carbonat darstellt,
(ii) einem aktiven Fe(-II)-Katalysatorkomplex,
(iii) mindestens einem Ligand,
(iv) mindestens einem Lösungsmittel und
(v) einem Nucleophil oder Pronucleophil.

2. Verfahren nach Anspruch 1, **dadurch gekennzeichnet, dass** das Carbonat das Strukturelement OC(O)OR umfasst, wobei R unverzweigte oder verzweigte Gruppe aufweist, substituiert oder unsubstituiert ist.

3. Verfahren nach Anspruch 2, **dadurch gekennzeichnet, dass** R eine Alkylgruppe und/oder Arylgruppe, vorzugsweise iso-Butyl, ist.

4. Verfahren nach den Ansprüchen 1, 2 oder 3, **dadurch gekennzeichnet, dass** es sich bei den Liganden um Phosphine handelt, bevorzugt um basische Phosphine, besonders bevorzugt um tertiäre Phosphine.

5. Verfahren nach einem der vorstehenden Ansprüche, **dadurch gekennzeichnet, dass** es sich bei dem Nucleophil oder Pronucleophil um ein C-Nucleophil handelt.

6. Verfahren nach Anspruch 5, **dadurch gekennzeichnet, dass** es sich bei dem C-Nucleophil um ein CH-acides Nucleophil oder Pronucleophil handelt.

7. Verfahren nach einem der Ansprüche 1, 2, 3 oder 4 **dadurch gekennzeichnet, dass** es sich bei dem Nucleophil oder Pronucleophil um ein N-, O- oder S-Nucleophil handelt.

8. Verfahren nach einem der vorstehenden Ansprüche, **dadurch gekennzeichnet, dass** es sich bei dem Lösungsmittel um DMF, THF oder MMP handelt.

9. Verfahren nach einem der vorstehenden Ansprüche, **dadurch gekennzeichnet, dass** der Fe(-II)-Katalysatorkomplex eine Reinheit von mindestens 80 % aufweist.

10. Verfahren nach Anspruch 9, **dadurch gekennzeichnet, dass** weniger als 25 Mol% des Katalysatorkomplexes [Bu4N][Fe(CO)3(NO)] eingesetzt wird.

11. Verfahren nach einem der vorstehenden Ansprüche, **dadurch gekennzeichnet, dass** die Aufreinigung des Fe(-II)-Katalysatorkomplexes mittels Umkristallisierung, vorzugsweise mit Methanol und Wasser, durchgeführt wird.

12. Verfahren nach einem der vorstehenden Ansprüche, **dadurch gekennzeichnet, dass** das Verfahren salzfrei durchgeführt wird.

13. Verwendung des Verfahrens nach einem der vorstehenden Ansprüche zur Synthese von Naturstoffen, insbesondere chiralen Naturstoffen.

14. Verwendung des Verfahrens nach einem der vorstehenden Ansprüche zur Synthese von Wirkstoffen.

15. Verwendung des Verfahrens nach einem der vorstehenden Ansprüche zur Synthese von nicht natürlichen Aminosäuren.

16. Verwendung des Verfahrens nach einem der vorstehenden Ansprüche für die asymmetrische Olefinierung.

17. Verwendung von [Bu4N][Fe(CO)3(NO)] zur Durchführung des Verfahrens nach einem der vorstehenden Ansprüche.

18. Eisen-Katalysatorkomplex erhältlich aus dem Verfahren nach Anspruch 11.

19. Eisen-Katalysatorkomplex erhältlich aus dem Verfahren nach Ansprüchen 1 und 4.
